# EUROPEAN PATENT SPECIFICATION

(11) **EP 1 855 650 B1**
(45) Date of publication and mention of the grant of the patent: **05.12.2012**
(21) Application number: 06707447.6
(22) Date of filing: 16.02.2006
(51) Int. Cl.: A61K 9/06, A61K 47/32, A61K 31/4164

(54) **AQUEOUS TOPICAL GEL OF HIGH STABILITY BASED ON METRONIDAZOLE AND METHOD OF PREPARATION**
WÄSSRIGES TOPISCHES GEL HOHER STABILITÄT AUF BASIS VON METRONIDAZOL UND HERSTELLUNGSVERFAHREN
GEL TOPIQUE AQUEUX A STABILITE ELEVEE, A BASE DE METRONIDAZOLE, ET PROCEDE DE PREPARATION

(30) Priority: 25.02.2005 FR 0501949; 15.04.2005 US 671635 P
(43) Date of publication of application: 21.11.2007
(73) Proprietor: Galderma S.A., 6330 Cham (CH)
(72) Inventor: ZARIF, Leila, F-06530 Peymeinade (FR); PEDRASSI, Gérald, F-83300 Draguignan (FR); BRZOKEWICZ, Alain, F-06560 VALBONNE (FR)
(74) Representative: Boulard, Denis
(86) International application number: PCT/EP2006/002074
(87) International publication number: WO 2006/089804

(56) References cited:
- WO-A-88/06888
- WO-A-89/06537
- WO-A-2004/017998
- US-A- 4 837 378
- US-A- 5 536 743
- US-A- 5 593 680
- US-A- 6 143 794
- US-B1- 6 258 816

## Description

The present invention relates to a novel method for preparing metronidazole-based aqueous gels which are useful in particular as topical dermatological compositions, especially for the treatment of dermatoses, such as rosacea.

Metronidazole, or 1-(2-hydroxyethyl)-2-methyl-5-nitroimidazole, is the compound of the formula (I) :

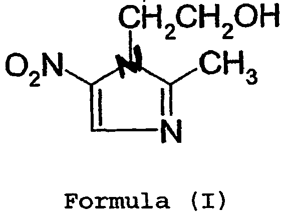

This compound and its mode of preparation are well known and have been described in particular in Patent US 2,944,061.

Metronidazole is an acknowledged antibacterial and antiparasitic agent useful for the treatment of many conditions. This compound is known in particular as being particularly effective in the treatment of skin disorders such as rosacea.

Rosacea is a chronic skin condition which affects mainly adults. It is a type of dermatosis with recurrent symptoms, including in particular erythemas, papules, pustules, rhinophymas and/or telangiectasias, which manifests itself mainly in the region of the nose, the cheeks and the forehead.

For the treatment of such conditions, metronidazole is preferably administered by the topical route. Indeed, administration by the systemic route, in particular by the oral route, leads, in most cases, to undesirable side effects, such as gastrointestinal intolerance or vaginitis, to which other chronic disorders may also be added in the case of a long-term administration.

Various topical formulations have been proposed for the topical administration of metronidazole, which are mainly oil-based compositions, in particular creams (oil-in-water emulsions) or ointments (in particular compositions based on petroleum jelly). In these compositions, metronidazole is dissolved in the oily phase. These oil-based compositions have the advantage of being able to contain large quantities of metronidazole in the solubilised state, available for topical application. However, they are found, in practice, to be poorly suited to dermatological use. Indeed, they require the presence of ingredients, in particular oils, emulsifiers or surfactants, which are found to exhibit comedogenic, acneigenic, drying and/or irritating properties for the skin. Furthermore, patients treated with compositions of this type often feel sensations of burning or urtication.

To avoid these problems, there have been proposed, in patent US 4,837,378, compositions in the form of aqueous gels which do not require the presence of comedogenic, acneigenic, drying and/or irritating agents present in the abovementioned oil-based compositions.

The compositions of US 4,837,378 comprise metronidazole in an aqueous phase gelled with a polymeric gelling agent, such as a polycarboxylated vinyl polymer. The method for preparing these compositions, as described in US 4,837,378, consists in mixing the gelling polymer with an aqueous solution of metronidazole.

The compositions of US 4,837,378 may additionally comprise additional agents, such as propylene glycol which improve the efficacy of administration of metronidazole, or preservatives such as methylparaben or propylparaben. Where appropriate, these additives are added to the preformed solution of metronidazole in water, and then the mixture obtained is mixed with the gelling polymer.

The aqueous gels of US 4,837,378 have numerous advantages, in addition to the fact that they do not comprise ingredients capable of leading to the side effects observed with the abovementioned oil-based compositions. In particular, they allow better control of application and a uniform distribution of the active ingredient. Furthermore, they act as prolonged-release systems, which gradually deliver the active ingredient topically, in a therapeutically effective amount for a prolonged period.

One aim of the present invention is to provide aqueous gels for the topical administration of metronidazole having the advantages of the compositions of US 4,837,378, but additionally having increased stability over time.

For the purposes of the present description, the expression "stability" of an aqueous metronidazole gel is understood to mean its capacity to retain its gel structure over a long period, with metronidazole mainly in the dissolved and non-crystallized state. It is considered that a gel is all the more stable if the period during which is retains its gel structure with metronidazole mainly in the dissolved state is long.

To this effect, according to a first aspect, the subject of the present invention is a method for preparing a stable aqueous gel based on metronidazole, comprising the successive steps consisting in:
(A) forming a solvent medium M comprising water and propylene glycol;
(B) dissolving the metronidazole in the solvent medium M thus obtained, this dissolution being optionally followed by dilution of the medium obtained by addition of water, whereby a solution S of metronidazole is obtained; and then
(C) mixing the solution S obtained with a gelling polymer having free carboxyl groups which are completely or partly neutralizable in form of carboxylates, in a sufficient quantity to ensure gelling of the composition.

For the purposes of the present description, the expression "metronidazole-based aqueous gel" is understood to mean a gelled aqueous phase, preferably monophasic, and containing metronidazole. Advantageously, this gel contains metronidazole as sole active ingredient with a cosmetic or pharmaceutical effect.

Preferably, the metronidazole is present in the gel in a therapeutically effective quantity. This metronidazole content may vary according to the application envisaged for the gel, but it advantageously remains between 0.25% and 1.0% by mass, and it is typically of the order of 0.75 to 0.8% by mass relative to the total mass of the aqueous gel.

In the context of the present invention, the inventors have demonstrated that, during the preparation of such an aqueous gel, a dissolution of the metronidazole in a medium initially containing water and propylene glycol, of the M medium type in step (B), makes it possible to obtain aqueous metronidazole gels having high stability. Unexpectedly, the work by the inventors has in particular made it possible to establish that the use of this specific step of dissolution in a propylene glycol-based medium makes it possible to obtain gels with increased stability compared to the gels obtained according to the methods of US 4,837,378, where the metronidazole is first dissolved in water, and where propylene glycol is introduced into the medium afterwards.

Even more surprisingly, this stabilization effect is found to be most particularly pronounced when the propylene glycol/water ratio by mass in the medium M formed in step (A) is between 2% and 15%, typically between 3 and 10%. Thus, the method of the invention requires in general much less propylene glycol than the method of US 4,837,378, where this compound is introduced in an amount of 7.9% by mass into the metronidazole dispersion. This use of propylene glycol in a limited quantity constitutes another advantage of the method of the invention, in particular from an economic point of view.

Without wishing to be bound by a particular theory, it seems that it is possible to see that the stabilizing effect observed by the inventors is at least partly due to the fact that a mixture of water and propylene glycol allows a very good solubilization of metronidazole, in particular when the water and the propylene glycol are present in the abovementioned advantageous ranges, whereas a lower solubilization is obtained when the propylene glycol is introduced after the dissolution of the metronidazole in water, even when the propylene glycol is post-introduced in large quantities as in the method of US 4,837,378. In this regard, the inventors have demonstrated that while metronidazole has a solubility in water of the order of 0.78%, water/propylene glycol mixtures can bring about higher solubilities.

According to an advantageous embodiment of the method of the invention, medium M formed in step (A) comprises a preservative in addition to the water and the propylene glycol, this preservative being preferably present in a quantity effective to inhibit microbial growth in the gel during its storage. Advantageously, this preservative is chosen from the paraben family. In particular, a mixture of methylparaben and of propylparaben is found to be particularly advantageous to this effect. In particular, in these amounts, the presence of preservatives such as parabens in medium M also makes it possible, in some cases, to improve the solubilization of metronidazole during step (B), and it also leads to an improvement in the stability of the gel obtained.

Medium M most often consists of a mixture of water, propylene glycol and optionally preservatives of the abovementioned type, excluding any other compound. Nevertheless, the presence of other chemical species is not excluded insofar as they do not hamper effective solubilization of metronidazole in step (B).

Thus, according to a particular embodiment, the medium of step (A) may for example comprise ethylene diamine tetraacetic acid (EDTA) or one of its salts as additional ingredient, although it is most often preferable for this acid to be introduced after the dissolution of metronidazole in step (B).

EDTA, which is commonly used in dermatological compositions, is useful in particular for chelating metal cations which may be present as impurities in the composition, which makes it possible in particular to avoid undesirable side effects in some patients. EDTA also inhibits the phenomena of browning of the composition which can occur during storage of the gel, in particular when the pH of the gel is of the order of 3.5 to 5.4. The gel prepared according to the method of the present invention advantageously contains EDTA, preferably in an amount of 0.01 to 0.1% by mass, and typically in an amount of the order of 0.05% by mass.

Regardless of the exact composition of the medium M formed in step (A), it is preferable, in general, that, in step (B), the dissolution of metronidazole in this medium M is carried out at a temperature greater than 40°C, this temperature being advantageously at least 45°C, and most preferably at least 50°C, in particular in order to obtain optimum dissolution of the metronidazole. It is preferable, moreover, that this temperature remains less than 70°C, and preferably less than or equal to 60°C, in particular for economic reasons. Thus, this temperature is typically of the order of 50 to 55°C.

Moreover, in step (B), the metronidazole is introduced into medium M with a mass ratio (metronidazole/medium M) which is most often between 0.5 and 2.5, for example between 0.8 and 2.2, typically between 1 and 2.

According to a particular embodiment, step (B) simply consists in dissolving metronidazole in medium M prepared in step (A).

Another variant of step (B) consists in dissolving, in a first instance, the metronidazole in medium M prepared in step (A), and then in diluting the metronidazole solution by adding water. According to this variant, the water is in general introduced alone as dilution medium, but it is not impossible, according to a particular embodiment, for the water for dilution to be introduced in the form of an aqueous solution containing water-soluble compounds, for example EDTA.

Regardless of the embodiment of step (B), this step leads to a solution S of metronidazole in aqueous medium being obtained. This solution S has, in general, a metronidazole concentration greater than 0.5% by mass, and it is typically between 0.7 and 2.5% by mass, relative to the total mass of solution S. In this solution, the metronidazole is mainly in solubilized form. Thus, in general, in solution S obtained at the end of step (B), less than 1%, and most often less than 0.5%, or even less than 0.1%, of the total mass of metronidazole is in the insolubilized state.

Step (C) of mixing solution S with the gelling polymer may be carried out by any means known to persons skilled in the art. Thus, the gelling agent may in particular be simply introduced into solution S. Alternatively, the polymer may first be mixed with water, and optionally with other water-soluble compounds such as EDTA or pH-regulating agents such as sodium hydroxide, to form a pre-gel, this pre-gel then being mixed with solution S.

The gelling polymer used in step (C) may be any polymer suitable for gelling an aqueous phase in order to form a gel of uniform structure. This is preferably a water-dispersible polymer, having high affinity for water, this polymer being preferably a polymer having free carboxyl groups which are completely or partly neutralizable in the form of carboxylates with a base. Particularly advantageous gelling polymers in step (C) are vinyl polymers with water-dispersible carboxylate groups, in particular poly(acrylic acids) neutralized with a base. Most particularly advantageous are polymers having a molecular mass of the order of 1 250 000 to 4 000 000. Thus, suitable poly(acrylic acids) are for example the poly(acrylic acids) crosslinked with compounds of the polyalkenyl polyether type, such as carbomers, such as the polymers available from the company Goodrich under the trade name Carbopol 934, 940 or 941, Carbopol 940 being most particularly preferred.

The neutralization of the carboxyl groups in the gelling polymers described above may be carried out by adding to the gel a base, for example aqueous ammonia, NaOH or organic amines, such as alkylamines (for example methylamine, ethylamine) or alternatively dialkylamines, trialkylamines, alkanolamines or dialkanolamines. In this context, it should be noted that the metronidazole itself is sufficiently basic to bring about partial neutralization of the acid groups and thus bring about at least an onset of gelling. The final pH of the gel may vary widely according to the application envisaged. However, most often, this pH is of the order of 3 to 6.9, and preferably between 4 and 5.5.

The gelling polymer is introduced in step (C) in a sufficient quantity to lead in fine to a gel being obtained. Advantageously, this quantity is sufficient to produce a viscosity suitable for topical application of the gel, in particular for dermatological applications. In particular, to obtain such a viscosity, the gelling polymer is advantageously introduced in step (C) in a quantity such that its final content in the gel is between 0.2% and 7.0% by mass, preferably between 0.5% and 1.5% by mass, relative to the total mass of the gel, this content being typically of the order of 0.6% by mass.

According to an advantageous embodiment, steps (A) to (B) of the method of the invention consist in:
(A1) forming a medium M comprising water and propylene glycol, preferably in combination with preservatives and EDTA;
(B1) dissolving the metronidazole in the aqueous medium M thus formed, so as to form a solution S of metronidazole; and then
(C1) introducing into the solution S obtained a gelling polymer in a sufficient quantity to bring about gelling of the composition.

According to this embodiment, steps (A1) to (C1) advantageously consist in:
(a1) forming a medium M comprising from 96% to 98% of water, from 2% to 5% (preferably between 2.5 and 3.5%) of propylene glycol. From 0 to 2% (preferably from 0.5% to 1.5%) of preservatives (typically a mixture of methylparaben and propylparaben) and from 0 to 2% (preferably of the order of 0.8 to 1.2%, and typically of the order of 1%) of EDTA;
(b1) dissolving metronidazole in the aqueous medium M thus formed, with a metronidazole/medium M ratio by mass of between 0.5% and 1% (advantageously of the order of 0.7 to 0.9%) so as to form a solution S of metronidazole; and then (c1) introducing into the solution S obtained a gelling polymer carrying free carboxyl groups neutralized in the from of carboxylates, with a gelling polymer/solution S ratio by mass of between 0.5% and 1%, typically between 0.6% and 0.8%.

According to another advantageous embodiment, steps (A2) to (C2) of the method of the invention consist in:
(A2) forming a medium M comprising water and propylene glycol, preferably in combination with preservatives;
(B2) dissolving the metronidazole in the aqueous medium M1 thus formed, so as to form a solution S' of metronidazole, and then adding water to this solution S', whereby a solution S of metronidazole is obtained; and then
(C2) mixing the solution S obtained with a gelled aqueous phase containing a gelling polymer, in a sufficient quantity to bring about gelling of the entire composition, preferably in combination with EDTA.

According to an advantageous variant, these steps (A2) to (C2) consist in:
(a2) forming a medium M comprising 90% to 95% of water, 5% to 10% (preferably between 7% and 8%) of propylene glycol, and from 0 to 0.5% (preferably from 0.2% to 0.4%) of preservatives (preferably a mixture of methylparaben and propylparaben);
(b2) dissolving metronidazole in the aqueous medium M thus formed, with a metronidazole/medium M ratio by mass of between 1.5% and 2.5% (advantageously of the order of 1.9% to 2.2%) so as to form a solution S' of metronidazole, and then adding water to this solution S' so as to obtain a solution S of metronidazole having a metronidazole content of 1% to 2.2%, preferably between 1.8% to 2.1% by mass (typically of the order of 1.9% by mass); and then
(c2) mixing the solution S obtained with a gelled aqueous phase containing 98% to 99.5% of water, 0.5% to 1.5% (preferably 0.8% to 1.1%) of a gelling polymer carrying free carboxyl groups neutralized in the form of carboxylates, with a gelling aqueous phase/solution S ratio by mass of between 1.4:1 and 1.8:1, typically between 1.5:1 and 1.7:1.

Regardless of its embodiment, the method of the invention makes it possible to obtain metronidazole-based aqueous gels which have a high stability during their storage. These particularly stable compositions constitute a second subject of the present invention.

The subject of the present invention also relates to compositions, in particular aqueous gels, which can be obtained according to one of the methods described above.

These compositions, more particularly these aqueous gels, are in particular suitable for the preparation of a medicament intended for application by the topical route for the prophylactic or therapeutic treatment of skin conditions, in particular in human beings.

In the context of the present invention, these compositions, in particular these gels, are in particular suitable for the treatment of rosacea, or of various forms of acne, such as acne vulgaris, acne conglobata or nodulocystic acne, or certain types of dermatitis, such as perioral or seborrhoeic dermatitis.

These various uses of the gels which can be obtained according to the abovementioned steps (A) to (C) further constitute another aspect of the present invention.

Various aspects and advantages of the invention will emerge from the illustrative examples set out below.

### Example 1 - Method for preparing an aqueous gel G1 based on metronidazole

An aqueous gel G1 based on metronidazole was prepared under the following conditions:

### • Preparation of a solvent medium based on water and propylene glycol

In a manufacturing tank kept at 50°C +/- 3°C, 900 kg of purified water, 30 kg of propylene glycol and 0.5 kg of EDTA (ethylenediaminetetraacetic acid) were mixed. The mixing was carried out with the aid of a scraper at 25 revolutions per minute and a turbine at 1000 revolutions per minute, with a mixing period of 10 minutes.

0.8 kg of methylparaben and 0.2 kg of propylparaben were then added to the medium. The medium was then homogenized with the aid of a scraper at 25 revolutions per minute and a turbine at 3000 revolutions per minute, under a -0.3b vacuum and at a temperature of 50 +/- 3°C for 20 minutes.

A homogeneous solvent medium based on water and propylene glycol was thus obtained.

### • Dissolution of metronidazole

7.5 kg of metronidazole were introduced into the solvent medium thus prepared. The dissolution was carried out while the medium was kept at 50°C +/- 3°C, with stirring with the aid of a scraper at 25 revolutions per minute and a turbine at 1000 revolutions per minute, under a -0.3b vacuum, for 15 minutes.

A solution (s1) of metronidazole at about 0.8% by mass was thus obtained.

### • Gelling

The solution (s1) obtained was gelled by adding 6.5 kg of Carbomer 940 (marketed by Goodrich), in a dry form. The gelling agent was introduced gradually, with stirring with the aid of a scraper at 25 revolutions per minute and a turbine at 3000 revolutions per minute, for 30 minutes.

### Example 2 - Method for preparing an aqueous gel G2 based on metronidazole

An aqueous gel G2 based on metronidazole was prepared under the following conditions:

### • Preparation of a solvent medium based on water and propylene glycol

A mixture of 1.04 kg of methylparaben and 0.26 kg of propylparaben was gradually added to 36 kg of propylene glycol, with stirring. The stirring was maintained until complete dissolution of the methylparaben and propylparaben had been obtained.

The medium thus obtained was introduced into 429 litres of purified water, kept stirred in a 700 litre jacketed reactor, at a temperature of between 50 and 55°C. The mixing was carried out with the aid of a dispersing device.

A homogeneous solvent medium based on water and propylene glycol was thus obtained.

### • Dissolution of metronidazole

9.75 kg of metronidazole were introduced into the solvent medium thus prepared. The dissolution was carried out in the jacketed reactor and with the aid of the dispersing device of the preceding step, while the medium was kept between 50 and 55°C.

A solution of metronidazole (S2) at about 2% by mass was thus obtained.

### • Gelling

In a 1500 litre reactor, 772 kg of purified water were mixed with 0.65 kg of EDTA (ethylenediaminetetraacetic acid) and 7.8 kg of Carbomer 940 NF (Goodrich). The mixing was carried out with stirring at 850-1100 revolutions per minute, for 60 minutes.

A solution of 1.3 kg of NaOH in 13 litres of water was then introduced into the medium. The medium was then subjected to stirring at 30 revolutions per minute for 90 minutes.

At the end of the stirring, an aqueous phase gelled with Carbomer 940 NF was obtained.

This gelled phase was mixed with the solution (s2) of metronidazole obtained above, at 55°C. The mixture was prepared by introducing the solution (s1) into the gelled aqueous phase, allowing the medium to cool to 33°C, and then mixing with coaxial stirring at 30 revolutions per minute for 120 minutes.

At the end of the stirring, a gel G2 based on metronidazole was obtained.

### Example 3 - Method for preparing an aqueous gel G3 based on metronidazole

An aqueous gel based on metronidazole was prepared under the following conditions:

### • Preparation of a solvent medium based on water and propylene glycol

60 kg of propylene glycol, 1.6 kg of methylparaben and 0.4 kg of propylparaben were mixed.

The mixture thus obtained was introduced into 660 litres of purified water, kept stirred at 1000 revolutions per minute in a reactor kept at a temperature of 53°C +/- 2°C.

A homogeneous solvent medium based on water and propylene glycol was thus obtained.

### • Dissolution of metronidazole

15 kg of metronidazole were introduced into the solvent medium thus prepared. The dissolution was carried out while the medium was kept at 53°C +/- 2°C, with a turbine stirring at 1000 revolutions per minute, with a coaxial at 30 revolutions per minute, for 5 minutes.

A solution of metronidazole (S3) at about 2% by mass was thus obtained.

### • Gelling

In a 2000 litre reactor, 1148 kg of purified water were mixed with 1 kg of EDTA (ethylenediaminetetraacetic acid), with turbine stirring at 1000 revolutions per minute, with a coaxial at 30 revolutions per minute, for 5 minutes. 12 kg of Carbomer 940 NF (Goodrich) were then added to this medium under a -0.4b vacuum. The mixing was carried out with stirring at 1000 revolutions per minute for 60 minutes.

A solution of 2 kg of NaOH in 20 litres of purified water was then introduced into the medium. The medium was then subjected to stirring at 1000 revolutions per minute, under a -0.6b vacuum, for 90 minutes.

At the end of the stirring, an aqueous phase gelled with the Carbomer 940 NF was obtained.

The solution (s1) of metronidazole prepared above was mixed with this gelled phase. The mixture was prepared by introducing the solution (s3) prepared above into the gelled aqueous phase, allowing the medium to cool to 31°C +/- 2°C, and then mixing, with coaxial stirring at 30 revolutions per minute under a -0.8b vacuum, for 120 minutes.

At the end of the stirring, a gel G3 based on metronidazole was obtained.

## Claims

1. Method for preparing a stable aqueous gel based on metronidazole, comprising the successive steps consisting in:
(A) forming a solvent medium M comprising water and propylene glycol;
(B) dissolving the metronidazole in this solvent medium M, this dissolution being optionally followed by dilution of the medium obtained by addition of water, whereby a solution S of metronidazole is obtained; and then
(C) mixing the solution S obtained with a gelling polymer having free carboxyl groups which are completely or partly neutralizable in form of carboxylates, in a sufficient quantity to ensure gelling of the composition.

2. Method according to Claim 1, where the propylene glycol/water ratio by mass in the medium M formed in step (A) is between 2% and 15%.

3. Method according to Claim 1 or according to Claim 2, where the medium M formed in step (A) additionally contains a preservative.

4. Method according to Claim 3, where the preservative is a mixture of methylparaben and propylparaben.

5. Method according to any one of the preceding claims, where, in step (B), the dissolution of metronidazole in the medium M is carried out at a temperature of between 40 and 60°C.

6. Method according to any one of the preceding claims, where, in step (B), the metronidazole is introduced into the medium M with a ratio by mass (metronidazole/medium M) of between 0.5 and 2.5%.

7. Method according to Claim 6, in which the solution of metronidazole S obtained at the end of step (B) has a metronidazole concentration greater than 0.5% by mass, relative to the total mass of the aqueous solution.

8. Method according to any one of the preceding claims, where, in the solution of metronidazole S obtained at the end of step (B), less than 1% of the total mass of metronidazole is in the insolubilized state.

9. Method according to Claim 1, comprising the steps consisting in:
(a1) forming a medium M comprising from 96% to 98% of water, from 2% to 5% of propylene glycol, from 0 to 2% of preservatives and from 0 to 2% of EDTA;
(b1) dissolving metronidazole in the aqueous medium M thus formed, with a metronidazole/medium M ratio by mass of between 0.5% and 1% so as to form a solution S of metronidazole; and then
(c1) introducing into the solution S obtained a gelling polymer carrying free carboxyl groups neutralized in the form of carboxylates, with a gelling polymer/solution S ratio by mass of between 0.5% and 1%.

10. Method according to any one of Claims 1 to 9, comprising the steps consisting in:
(A2) forming a medium M comprising water and propylene glycol;
(B2) dissolving the metronidazole in the aqueous medium M thus formed, so as to form a solution S' of metronidazole, and then adding water to this solution S', whereby a solution S of metronidazole is obtained; and then
(C2) mixing the solution S obtained with a gelled aqueous phase containing a gelling polymer carrying free carboxyl groups neutralized in the form of carboxylates , in a sufficient quantity to bring about gelling of the entire composition.

11. Method according to Claim 10, comprising the steps consisting in:
(a2) forming a medium M comprising 90% to 95% of water, 5% to 10% of propylene glycol, and from 0 to 0.5% of preservatives;
(b2) dissolving metronidazole in the aqueous medium M thus formed, with a metronidazole/medium M ratio by mass of between 1.5% and 2.5% so as to form a solution S' of metronidazole, and then adding water to this solution S' so as to obtain a solution S of metronidazole having a metronidazole content of 1% to 2.2%; and then
(c2) mixing the solution S obtained with a gelled aqueous phase containing 98% to 99.5% of water, 0.5% to 1.5% of a gelling polymer carrying free carboxyl groups neutralized in the form of carboxylates, with a gelling aqueous phase/solution S ratio by mass of between 1.4:1 and 1.8:1.

## Patentansprüche

1. Verfahren zum Herstellen eines stabilen wässrigen Gels basierend auf Metronidazol, umfassend die aufeinanderfolgenden Schritte bestehend aus:
(A) Bilden eines Lösemittelmediums M, umfassend Wasser und Propylenglycol;
(B) Lösen von Metronidazol in diesem Lösemittelmedium M, wobei dieser Auflösung wahlweise die Verdünnung des Mediums folgt, das durch die Zugabe von Wasser erhalten wird, wodurch eine Lösung S aus Metronidazol erhalten wird; und dann
(C) Mischen der erhaltenen Lösung S mit einem Gelierpolymer mit freien Carboxylgruppen, die vollständig oder teilweise in Form von Carboxylaten neutralisierbar sind, in einer ausreichenden Menge, um die Gelierung der Zusammensetzung sicherzustellen.

2. Verfahren nach Anspruch 1, wobei das Propylenglycol-Wasser-Massenverhältnis in dem Medium M, das in Schritt (A) gebildet wurde, zwischen 2 % und 15 % beträgt.

3. Verfahren nach Anspruch 1 oder nach Anspruch 2, wobei das Medium M, das in Schritt (A) gebildet wurde, zusätzlich ein Konservierungsmittel enthält.

4. Verfahren nach Anspruch 3, wobei das Konservierungsmittel eine Mischung aus Methylparaben und Propylparaben ist.

5. Verfahren nach einem der vorhergehenden Ansprüche, wobei in Schritt (B) die Auflösung von Metronidazol in dem Medium M bei einer Temperatur von zwischen 40 und 60°C durchgeführt wird.

6. Verfahren nach einem der vorhergehenden Ansprüche, wobei in Schritt (B) das Metronidazol in das Medium M mit einem Massenverhältnis (Metronidazol zu Medium M) von zwischen 0,5 und 2,5 % eingeführt wird.

7. Verfahren nach Anspruch 6, wobei die Lösung von Metronidazol S, die am Ende von Schritt (B) erhalten wird, eine Metronidazolkonzentration von mehr als 0,5 Masseprozent in Bezug auf die Gesamtmasse der wässrigen Lösung aufweist.

8. Verfahren nach einem der vorhergehenden Ansprüche, wobei in der Lösung von Metronidazol S, die am Ende von Schritt (B) erhalten wird, weniger als 1 % der Gesamtmasse von Metronidazol in ungelöstem Zustand vorliegt.

9. Verfahren nach Anspruch 1, umfassend die Schritte bestehend aus:
(a1) Bilden eines Mediums M, umfassend von 96 % bis 98 % Wasser, von 2 % bis 5 % Propylenglycol, von 0 bis 2 % Konservierungsmittel und von 0 bis 2 % EDTA;
(b1) Lösen von Metronidazol in dem wässrigen Medium M, das auf diese Weise gebildet wird, mit einem Metronidazol-Medium M-Massenverhältnis von zwischen 0,5 % und 1 %, sodass eine Lösung S aus Metronidazol gebildet wird; und danach
(c1) Einführen eines Gelierpolymers in die erhaltene Lösung S, das freie Carboxylgruppen trägt, die in Form von Carboxylaten neutralisiert wurden, und mit einem Gelierpolymer-Lösung S-Massenverhältnis von zwischen 0,5 % und 1 %.

10. Verfahren nach einem der vorhergehenden Ansprüche 1 bis 9, umfassend die Schritte bestehend aus:
(A2) Bilden eines Mediums M, umfassend Wasser und Propylenglycol;
(B2) Lösen von Metronidazol in dem auf diese Weise gebildeten wässrigen Medium M, um eine Lösung S' von Metronidazol zu bilden, und danach Zugeben von Wasser zu dieser Lösung S', wobei eine Lösung S von Metronidazol erhalten wird; und danach
(C2) Mischen der erhaltenen Lösung S mit einer gelierten wässrigen Phase, enthaltend ein Gelierpolymer, das freie Carboxylgruppen trägt, die in Form von Carboxylaten neutralisiert wurden, in einer ausreichenden Menge, um die Gelierung der gesamten Zusammensetzung zu bewirken.

11. Verfahren nach Anspruch 10, umfassend die Schritte bestehend aus:
(a2) Bilden eines Mediums M, umfassend 90 % bis 95 % Wasser, 5 % bis 10 % Propylenglycol und von 0 bis 0,5 % Konservierungsmittel;
(b2) Lösen von Metronidazol in dem auf diese Weise gebildeten wässrigen Medium M, mit einem Metronidazol-Medium M-Massenverhältnis von zwischen 1,5 % und 2,5 % zum Bilden einer Lösung S' von Metronidazol und dann Zugeben von Wasser zu dieser Lösung S', um eine Lösung S von Metronidazol mit einem Metronidazol-Gehalt von 1 % bis 2,2 % zu erhalten; und dann
(c2) Mischen der erhaltenen Lösung S mit einer gelierten wässrigen Phase, enthaltend 98 % bis 99,5 % Wasser, 0,5 % bis 1,5 % eines Gelierpolymers, das freie Carboxylgruppen trägt, die in Form von Carboxylaten neutralisiert werden, mit einem gelierten wässrigen Phasen-Lösung S-Massenverhältnis von zwischen 1,4 : 1 und 1,8 : 1.

## Revendications

1. Procédé de préparation d'un gel aqueux stable à base de métronidazole, comprenant les étapes successives consistant à :
(A) former un milieu solvant M comprenant de l'eau et du propylène glycol ;
(B) dissoudre le métronidazole dans ce milieu solvant M, cette dissolution étant éventuellement suivie par une dilution du milieu obtenu par addition d'eau, ce par quoi on obtient une solution S de métronidazole ; puis
(C) mélanger la solution S obtenue avec un polymère gélifiant ayant des groupements carboxyliques libres qui sont entièrement ou partiellement neutralisables sous forme de carboxylates, en une quantité suffisante pour assurer la gélification de la composition.

2. Procédé selon la revendication 1, où le rapport massique propylène glycol/eau dans le milieu M formé dans l'étape (A) est compris entre 2% et 15%.

3. Procédé selon la revendication 1 ou selon la revendication 2, où le milieu .M formé dans l'étape (A) contient en outre un agent conservateur.

4. Procédé selon la revendication 3, où l'agent conservateur est un mélange de méthylparabène et de propylparabène.

5. Procédé selon l'une quelconque des revendications précédentes, où, dans l'étape (B), la dissolution du métronidazole dans le milieu M est réalisée à une température comprise entre 40 et 60°C.

6. Procédé selon l'une quelconque des revendications précédentes, où, dans l'étape (B), le métronidazole est introduit dans le milieu M avec un rapport massique (métrodinazole/milieu M) compris entre 0,5 et 2,5 %.

7. Procédé selon la revendication 6, dans laquelle la solution de métronidazole S obtenue à l'issue de l'étape (B) a une concentration en métronidazole supérieure à 0,5 % en masse, par rapport à la masse totale de la solution aqueuse.

8. Procédé selon l'une quelconque des revendications précédentes, où, dans la solution de métronidazole S obtenue à l'issue de l'étape (B), moins de 1% de la masse totale du métronidazole est à l'état insolubilisé.

9. Procédé selon la revendication 1, comprenant les étapes consistant à :
(a1) former un milieu M comprenant de 96% à 98% d'eau, de 2% à 5% de propylène glycol, de 0 à 2% d'agents conservateurs, et de 0 à 2% d'EDTA ;
(b1) dissoudre du métronidazole dans le milieu aqueux M ainsi formé, avec un rapport massique métronidazole/milieu M compris entre 0,5% et 1%, de façon à former une solution S de métronidazole ; puis (c1) introduire dans la solution S obtenue un polymère gélifiant porteur de groupements carboxyliques libres neutralisés sous forme de carboxylates, avec un rapport massique polymère gélifiant/solution S compris entre 0,5% et 1%.

10. Procédé selon l'une quelconque des revendications 1 à 9, comprenant les étapes consistant à :
(A2) former un milieu M comprenant de l'eau et du propylène glycol ;
(B2) dissoudre le métronidazole dans le milieu aqueux M ainsi formé, de façon à former une solution S' de métronidazole, puis ajouter de l'eau à cette solution S', ce par quoi on obtient une solution S de métronidazole ; puis
(C2) mélanger la solution S obtenue à une phase aqueuse gélifiée contenant un polymère gélifiant porteur de groupements carboxyliques libres neutralisés sous forme de carboxylates, en une quantité suffisante pour assurer la gélification de la totalité de la composition.

11. Procédé selon ta revendication 10, comprenant les étapes consistant à :
(a2) former un milieu M comprenant 90% à 95% d'eau, 5% à 10% de propylène glycol, et de 0 à 0,5% d'agents conservateurs ;
(b2) dissoudre du métronidazole dans le milieu aqueux M ainsi formé, avec un rapport massique métronidazole/milieu M compris entre 1,5% et 2,5% de façon à former une solution S' de métronidazole, puis ajouter de l'eau à cette solution S' de façon à obtenir une solution S de métronidazole ayant une teneur en métronidazole de 1% à 2,2% ; puis
(c2) mélanger la solution S obtenue à une phase aqueuse gélifiée contenant 98% à 99,5% d'eau, 0,5% à 1,5% d'un polymère gélifiant porteur de groupements carboxyliques libres neutralisés sous forme de carboxylates, avec un rapport massique phase aqueuse gélifiante/solution S compris entre 1,4 1 et 1,8 : 1.
